(19) Europäisches Patentamt
European Patent Office
Office européen des brevets

(11) **EP 4 094 685 B1**

(12) **EUROPEAN PATENT SPECIFICATION**

(45) Date of publication and mention
of the grant of the patent:
**03.01.2024 Bulletin 2024/01**

(51) International Patent Classification (IPC):
***A61B 5/12*** *(2006.01)*

(21) Application number: **22175300.7**

(52) Cooperative Patent Classification (CPC):
**A61B 5/125**

(22) Date of filing: **25.05.2022**

(54) **SPECTRO-TEMPORAL MODULATION DETECTION TEST UNIT**

SPEKTRO-TEMPORALE MODULATIONSDETEKTIONSEINHEIT

UNITÉ D'ESSAI DE DÉTECTION DE MODULATION SPECTRO-TEMPORALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.05.2021 EP 21176255**

(43) Date of publication of application:
**30.11.2022 Bulletin 2022/48**

(60) Divisional application:
**23212259.8**

(73) Proprietor: **Interacoustics A/S
5500 Middelfart (DK)**

(72) Inventors:
• **ZAAR, Johannes
2765 Smørum (DK)**
• **SANCHEZ-LOPEZ, Raul
5500 Middelfart (DK)**
• **LAUGESEN, Søren
5500 Middelfart (DK)**

(74) Representative: **Demant
Demant A/S
Kongebakken 9
2765 Smørum (DK)**

(56) References cited:
• **LOTFI YOUNES ET AL: "Spectro-temporal modulation detection and its relation to speech perception in children with auditory processing disorder", INTERNATIONAL JOURNAL OF PEDIATRIC OTORHINOLARYNGOLOGY, ELSEVIER, AMSTERDAM, NL, [Online] vol. 131, 3 January 2020 (2020-01-03), XP086088384, ISSN: 0165-5876, DOI: 10.1016/J.IJPORL.2020.109860 [retrieved on 2020-01-03]**
• **NASSIRI REZA ET AL: "Illusory spectrotemporal ripples created with binaurally correlated noise", THE JOURNAL OF THE ACOUSTICAL SOCIETY OF AMERICA, AMERICAN INSTITUTE OF PHYSICS FOR THE ACOUSTICAL SOCIETY OF AMERICA, NEW YORK, NY, US, [Online] vol. 123, no. 4, 20 March 2008 (2008-03-20), pages 92-98, XP012111127, ISSN: 0001-4966, DOI: 10.1121/1.2800893 [retrieved on 2021-10-26]**
• **ZAAR JOHANNES ET AL: "Investigating the relationship between spectro-temporal modulation detection, aided speech perception, and directional noise reduction preference in hearing-impaired listeners", PROCEEDINGS OF THE INTERNATIONAL SYMPOSIUM ON AUDITORY AND AUDIOLOGICAL RESEARCH , vol. 7 29 April 2020 (2020-04-29), pages 181-188, XP055798199, Retrieved from the Internet: URL:https://proceedings.isaar.eu/index.php /isaarproc/article/view/2019-22/386 [retrieved on 2021-10-26]**

EP 4 094 685 B1

**Description**

SUMMARY

The present application relates to a spectro-temporal modulation (STM) detection test unit. The present application further relates to an STM detection test system comprising an STM detection test unit and an auxiliary device.

The present application further relates to a method.

LOTFI YOUNES ET AL "Spectra-temporal modulation detection and its relation to speech perception in children with auditory processing disorder", INTERNATIONAL JOURNAL OF PEDIATRIC OTORHINOLARYNGOLOGY, ELSEVIER, AMSTERDAM, NL, vol. 131, 3 January 2020 relates to that poor speech perception in noise is one of the most common complaints reported for children with auditory processing disorder (APD). APD is defined as a deficit in perceptual processing of acoustic information in the auditory system in which decreased spectro-temporal resolution may also contribute. Since the recognition of spoken message in the context of other sounds, is based on the processing of auditory spectro-temporal modulations, the assessment of spectro-temporal modulations sensitivity can evaluate the listener's ability to retrieve and integrate speech segments covered by noise. Therefore, the purpose of this study was to examine spectro-temporal modulation (STM) detection and its relation to speech perception in children with APD and to compare the results with aged-matched normally developed children.

NASSIRI REZA ET AL "Illusory spectrotemporal ripples created with binaurally correlated noise", THE JOURNAL OF THE ACOUSTICAL SOCIETY OF AMERICA, AMERICAN INSTITUTE OF PHYSICS FOR THE ACOUSTICAL SOCIETY OF AMERICA, NEW YORK, NY, US, vol. 123, no. 4, 20 March 2008, relates to that binaural disparities are the primary acoustic cues employed in sound localization tasks. However, the degree of binaural correlation in a sound serves as a complementary cue for detecting competing sound sources. Here a random chord stereogram (RCS) sound is developed that produces a salient pop-out illusion of a slowly varying ripple sound, even though the left and right ear sounds alone consist of noise-like random modulations. The quality and resolution of this percept is systematically controlled by adjusting the spectrotemporal correlation pattern between the left and right sounds. The prominence and limited time-frequency resolution for resolving the RCS suggests that envelope correlations are a dominant binaural cue for grouping acoustic objects.

Background

The STM detection test has received much interest lately as a simple, language-independent measure of supra-threshold hearing ability, and more specifically as a proxy for complicated aided speech-in-noise testing.

In the STM detection test, a spectro-temporally modulated probe sound is compared to an unmodulated reference sound, with an otherwise similar spectrum. The reference sound, or the carrier signal, is typically a broad-band noise signal (while alternative carriers have been considered).

By means of an adaptive rule, the degree of modulation (modulation depth) in the probe sound is varied until the patient's threshold of modulation-detection is reached. The degree of modulation at threshold is the result of the test.

High correlations between STM thresholds and speech reception thresholds (SRT) have been observed in several studies [1][2][3][4][5], particularly for STM stimulus parameters that are similar to those observed for real speech, e.g. temporal modulations around 4 Hz and spectral modulations around 2 cycles/octave. In basic terms, one may assume that the degree of modulation a listener needs to detect the modulation in the STM stimulus is directly related to the signal-to-noise ratio (SNR) that the listener requires (at a minimum) to understand speech in background noise.

A version of the STM test that has been optimized for clinical use, is named the Audible Contrast Threshold (ACT) test. In the current embodiment of the ACT test, the same signal is played to both ears of the listener, apart from ear-specific amplification based on the respective audiograms.

In keeping with the analogy between signal-to-noise-ratio (SNR) in a speech test and the degree of modulation in the STM/ACT test, this would correspond to a speech test where both ears receive identical speech and noise signals, which rarely happens in real life. In fact, it is well established that listeners benefit strongly from so-called "better-ear" listening in realistic speech reception tasks with spatially distributed sound sources. Depending on the spatial location of target speaker and interfering sound sources, the SNR may be higher in one of the two ears due to the head-shadow effect.

In the simplest case, this can take place in a long-term sense, where one ear consistently shows an advantageous SNR. In a more complex setting with several non-stationary interfering sounds, the better-ear effect may well involve integration of high-SNR "glimpses" from both ears that can be local both in time and frequency, also called binaural glimpsing [6] [7] [8] [9].

While most previous studies on STM detection have measured the two ears of each participant separately (e.g. as opposed to the ACT test where they are measured jointly), no reported attempt has been made to assess listener's ability to (i) use the "better ear" when suitable or to (ii) integrate complementary spectral, temporal, or spectro-temporal information across the two ears. These abilities are crucial for speech understanding in real life and knowing to what extent they are degraded in a hearing-aid user is of importance when determining the optimal type and adjustment of hearing-aid processing to be provided to that user, especially regarding the use of integrated processing between the two hearing aids for binaural hearing-aid users and the spatial acuity of the sound processing.

Accordingly, there is a need for determining a user's ability to use the "better ear" when suitable and/or to integrate complementary spectral, temporal, or spectro-temporal information across the two ears.

An STM detection test unit

In an aspect of the present application, an STM detection test unit is provided.

The STM detection test unit comprises a stimulus generation unit.

The stimulus generation unit comprises at least one output unit. The at least one output unit may be a two-channel output unit.

The output unit is configured to present a first probe stimulus to one ear of a user.

The output unit is configured to present a second probe stimulus to another ear of the user.

The second probe stimulus is different from the first probe stimulus.

The first and second probe stimulus are presented simultaneously.

The STM detection test unit comprises an analysis unit.

The analysis unit is configured to determine, in response to presenting the probe stimuli (formed by the first and second probe stimulus, which are played simultaneously to both ears), a modulation-detection threshold of the user.

In other words, the analysis unit may be configured to determine a modulation-detection threshold of the user at the provided probe stimuli.

To determine may comprise that the analysis unit detects a response of the user of whether the user perceives the presented probe stimuli.

For example, the STM detection test unit may comprise a response detection unit, which detects a response from the user and transmits said response to said analysis unit.

Said analysis unit may detect a psychophysical or electrophysiological response of the user. To determine may comprise that the analysis unit calculates a modulation-detection threshold of the user based on the detected and/or received response of the user of whether the user perceives the presented probe stimuli.

The stimulus generation unit is configured to generate the first probe stimulus based on a carrier signal with a spectro-temporal modulation added.

The stimulus generation unit is configured to generate the second probe stimulus based on a carrier signal with a spectro-temporal modulation added.

The stimulus generation unit may be configured to generate each of the first probe stimulus and/or the second probe stimulus based on a carrier signal. Thereby, the first probe stimulus and/or the second probe stimulus may be provided to the user without a spectro-temporal modulation.

The stimulus generation unit may be configured to generate each of the first probe stimulus and the second probe stimulus based on the carrier signal with the spectro-temporal modulations added.

In other words, the stimulus generation unit may be configured to generate each of the first probe stimulus and/or the second probe stimulus based on the carrier signal on which a spectro-temporal modulation pattern is imposed.

The carrier signal of the first probe stimulus may be different from or similar to the carrier signal of the second probe stimulus.

The spectro-temporal modulation of the first probe stimulus may be different from the spectro-temporal modulation of the second probe stimulus.

Thereby, an apparatus for determining a user's ability to use the "better ear" when suitable or to integrate complementary spectral, temporal, or spectro-temporal information across the two ears is provided.

The STM detection test unit may be configured to operate in a plurality of different modes. Each mode may be characterized by the spectro-temporal modulation of the first probe stimulus being different from the spectro-temporal modulation of the second probe stimulus. Being able to operate in a plurality of different modes provides that the STM detection test unit may be set to the specific test mode best suited. For example, when the user's ability to use the "better ear" is to be examined, the test mode designed (best suited) for determining the better ear ability of the user can be chosen, and similar considerations apply for other modes. The best suited mode may be used in combination with a reference mode, for example the standard ACT mode, to estimate the better-ear ability.

The spectro-temporal modulation of the first probe stimulus being different from the spectro-temporal modulation of the second probe stimulus may comprise that the degree of the spectro-temporal modulation of the first probe stimulus is different from the degree of the spectro-temporal modulation of the second probe stimulus.

Degree may refer to the magnitude of the spectro-temporal modulation (modulation depth). The spectro-temporal modulation of the first probe stimulus being different from the spectro-temporal modulation of the second probe stimulus may comprise that the occurrence of the spectro-temporal modulation of the first probe stimulus is different from the occurrence of the spectro-temporal modulation of the second probe stimulus.

Occurrence may refer to the spectral and/or temporal occurrence of the spectro-temporal modulation so that only at certain (e.g. alternating) time intervals and/or at specific (e.g. alternating) frequency bands a spectro-temporal modulation is provided.

The analysis unit may be configured to compare a modulation-detection threshold (Thresh_mode) of the user in response to the stimuli with a modulation-detection threshold of a reference mode or condition (i.e. a reference modulation-detection threshold, Thresh_ref). For example, the reference modulation-detection threshold may be measured (in a reference mode) prior to using the STM detection test unit for the other plurality of different modes. Thereby, one or more reference modulation-detection threshold(s) may be available during use of the STM detection test unit. Reference modulation-detection thresholds may be available for each of the plurality of different modes of the STM detection test unit.

For example, the STM detection test unit may comprise a memory unit, and the memory unit may be configured to store the one or more reference modulation-detection thresholds.

The reference mode for determining (one or more) reference modulation-detection threshold(s) may comprise one of:

- Presenting the combined probe stimuli of the chosen mode of the STM detection test unit to both ears of the user (e.g. a diotic STM detection in response to similar stimuli, such as what may be done in a standard ACT test) and determining the corresponding modulation-detection threshold of the user.
- Presenting the combined probe stimuli of the chosen mode of the STM detection test unit to both ears of a normal-hearing subject and determining the corresponding modulation-detection threshold of the normal-hearing subject.
- Presenting similar sparse spectro-temporally modulated probe stimuli (as defined by the mode of the STM detection test unit) to both ears of the user (as opposed to different, complementary patterns presented to the two ears). Thereby, it will be possible to distinguish between the monaural effects of making the modulation pattern sparse and binaural benefit of integrating complementary sparse modulation patterns across the two ears.

The type of reference modulation-detection threshold used in the comparison with the modulation-detection threshold of the user may depend on whether the user's ability to use the "better ear" or to integrate information is to be examined.

Thereby, the user's ability to binaurally coordinate the perception of audio signals may be defined as the difference between the reference modulation-detection thresholds obtained using the reference mode with respect to the modulation-detection thresholds obtained using the plurality of different modes of the STM detection test unit.

Comparing the modulation-detection thresholds may comprise that the analysis unit is configured to determine a difference value ($\Delta_{thresh}$) between the modulation-detection threshold of the user in response to the stimuli and the reference modulation-detection threshold. The difference value may be: $\Delta_{thresh} = \text{Thresh\_mode} - \text{Thresh\_ref}$

The analysis unit may be configured to compare the user's difference value ($\Delta_{thresh}$) to an average difference value ($\Delta_{thresh, ava}$) measured for a group of young normal hearing listeners (normative data) at a similar test mode (i.e. at a similar mode of the STM detection test unit). Thereby, it may be determined whether the user's ability to use the better ear and/or to integrate across ears is decreased/impaired.

For example:

$\Delta_{thresh} < \Delta_{thresh, ava}$: User is able to use the better ear and/or to integrate across ears

$\Delta_{thresh} > \Delta_{thresh, ava}$: Decreased/impaired ability to use the better ear and/or integration In other words, the comparison of the determined difference value to the average difference value may define a user's ability to use the "better ear" when suitable and/or to integrate complementary spectral, temporal, or spectro-temporal information across the two ears.

In other words, comparison of the determined difference value to the average difference value may define a user's ability to binaurally coordinate the perception of audio signals.

For example, when a difference value is below the average difference value, the user may be able to coordinate binaurally.

For example, when a difference value exceeds the average difference value, the user may be unable to coordinate binaurally.

For example, the average difference value may be stored in the memory of the STM detection test unit.

Generating the first probe stimulus and the second probe stimulus may comprise that the stimulus generation unit is configured to modulate the carrier signal of each of the first probe stimulus and the second probe stimulus by a modulator signal with an adjustable modulation depth parameter.

The modulation depth parameter may determine the degree of modulation.

The modulation depth parameter may equal a value in the interval [0,1].

For example, the stimuli may be designed so that the first probe stimulus and the second probe stimulus consists of a carrier signal C(t,f), which is multiplied with a modulator signal, M(t,f), where t and f represent time and frequency, respectively. The tracking variable in the test may be the modulation depth parameter, m, which controls the degree of modulation and assumes values in the interval [0,1]. Accordingly, the full stimuli S(t,f), may thus be defined as:

$$S(t,f) \quad = \quad C(t,f) \cdot (1 + m \cdot M(t,f))$$

Generating the first probe stimulus and the second probe stimulus may comprise that the stimulus generation unit is configured to reduce the modulation depth parameter of either the first probe stimulus or the second probe stimulus by a modulation reduction parameter.

The modulation reduction parameter may equal a value in the interval from 0 to m, where m is the modulation depth parameter.

For example, in a mode relating to better-ear selection, stimuli with a smaller resulting modulation depth, described by the parameter, m-$\delta$, may be presented to one of the two ears of the user:

$$S_1(t,f) \quad = \quad C_1(t,f) \cdot (1 + m \cdot M(t,f))$$

$$S_2(t,f) \quad = \quad C_2(t,f) \cdot (1 + (m-\delta) \cdot M(t,f))$$

$S_1$ and $S_2$ denote the stimuli played to left and right ear (randomly assigned), $\delta$ is a modulation reduction parameter between 0 and m that reduces the modulation depth parameter in $S_2$. $C_1$ and $C_2$ are the carriers played to the left and right ears. The carriers might be identical or differ in phase while maintaining the same spectrum and energy.

Generating the first probe stimulus and the second probe stimulus may comprise that the stimulus generation unit is configured to provide a mask on the modulator signal of each of the first probe stimulus and the second probe stimulus.

The mask may equal values in the interval [0,1].

The mask on the modulator signal of the first probe stimulus may provide a complementary STM pattern to the mask on the modulator signal of the second probe stimulus.

For example, in a mode relating to temporal, spectral, and/or spectro-temporal integration across the ears of the user, the modulator signal, M, may be multiplied with a mask:

$$S_1(t,f) \quad = \quad C_1(t,f) \cdot (1 + m \cdot \Gamma(t,f) \cdot M(t,f))$$

$$S_2(t,f) \quad = \quad C_2(t,f) \cdot (1 + m \cdot (1 - \Gamma(t,f)) \cdot M(t,f))$$

$S_1$ and $S_2$ denote the stimuli played to left and right ear (randomly assigned). The mask, $\Gamma(t,f)$, may contain values in the interval [0,1], such that 1-$\Gamma(t,f)$ yields the complementary pattern to $\Gamma(t,f)$. The design of the mask, $\Gamma(t,f)$, determines whether $S_1$ and $S_2$ alternate temporally, spectrally, or spectro-temporally. $C_1$ and $C_2$ are the carriers played to the left and right ears. The carriers might be identical or differ in phase while maintaining the same spectrum and energy.

The STM detection test unit comprises a headset.

The headset comprises a first output transducer of the output unit for presenting the first probe stimulus to one of the ears of a user.

The headset comprises a second output transducer of the output unit for presenting the second probe stimulus to another ear of the user.

The output unit may be a two-channel output unit.

A headset may refer to an in-the-ear, on-the-ear, or over-the-ear headset, earphone, etc., which is configured to introduce audio into the ears of the user.

The STM detection test unit may comprise one or more detectors.

The one or more detectors may comprise one or more electrodes.

The STM detection test unit may be configured to determine the modulation-detection threshold of the user based on the user responding to heard modulated stimuli (e.g. by means of a push button or touch pad).

The STM detection test unit may be configured to determine the modulation-detection threshold of the user based on detecting a psychophysical response of the user by one or more detectors.

The STM detection test unit may be configured to determine the modulation-detection threshold of the user based on detecting a physiological response of the user by the one or more electrodes.

An STM detection test system:

In an aspect of the present application, an STM detection test system is provided.

The STM detection test system may comprise an STM detection test unit as disclosed above. The STM detection test system may comprise an auxiliary device.

The STM detection test system may be adapted to establish a communication link between the STM detection test unit and the auxiliary device to provide that information (e.g. test results, control and status signals, possibly audio signals) can be exchanged or forwarded from one to the other.

The auxiliary device may comprise a remote control, a smartphone, or other portable electronic device.

The auxiliary device may be constituted by or comprise a remote control for controlling functionality and operation of the STM detection test unit.

A hearing aid:

In an aspect of the present application, the auxiliary device of the STM detection system is a hearing aid.

The hearing aid may be adapted for being located at or in an ear of a hearing-aid user, or for being fully or partially implanted in the head of a hearing aid user.

The hearing aid may comprise an input unit for receiving an input sound signal from an environment of a hearing aid user and providing at least one electric input signal representing said input sound signal.

The input unit may comprise an input transducer, e.g. a microphone, for converting an input sound to an electric input signal. The input unit may comprise a wireless receiver for receiving a wireless signal comprising or representing sound and for providing an electric input signal representing said sound. The wireless receiver may e.g. be configured to receive an electromagnetic signal in the radio frequency range (3 kHz to 300 GHz). The wireless receiver may e.g. be configured to receive an electromagnetic signal in a frequency range of light (e.g. infrared light 300 GHz to 430 THz, or visible light, e.g. 430 THz to 770 THz).

The hearing aid may comprise a processing unit.

The processing unit may comprise signal processing parameters to provide processed versions of said at least one electric input signal.

The signal processing parameters may be configured at least based on the determined difference value.

In an aspect of the present application, a hearing aid comprising signal processing parameters configured by the determined difference value is provided.

The hearing aid may be adapted to provide a frequency dependent gain and/or a level dependent compression and/or a transposition (with or without frequency compression) of one or more frequency ranges to one or more other frequency ranges, e.g. to compensate for a hearing impairment of a user.

The hearing aid may comprise an output unit for providing a stimulus perceived by the user as an acoustic signal based on a processed electric signal. The output unit may comprise a number of electrodes of a cochlear implant (for a CI type hearing aid) or a vibrator of a bone conducting hearing aid. The output unit may comprise an output transducer. The output transducer may comprise a receiver (loudspeaker) for providing the stimulus as an acoustic signal to the user (e.g. in an acoustic (air conduction based) hearing aid). The output transducer may comprise a vibrator for providing the stimulus as mechanical vibration of a skull bone to the user (e.g. in a bone-attached or bone-anchored hearing aid).

The hearing aid may comprise a directional microphone system adapted to spatially filter sounds from the environment, and thereby enhance a target acoustic source among a multitude of acoustic sources in the local environment of the user wearing the hearing aid. The directional system may be adapted to detect (such as adaptively detect) from which direction a particular part of the microphone signal originates. This can be achieved in various different ways as e.g. described in the prior art. In hearing aids, a microphone array beamformer is often used for spatially attenuating background noise sources. Many beamformer variants can be found in literature. The minimum variance distortionless response (MVDR) beamformer is widely used in microphone array signal processing. Ideally, the MVDR beamformer keeps the signals from the target direction (also referred to as the look direction) unchanged, while attenuating sound signals from other directions maximally. The generalized sidelobe canceller (GSC) structure is an equivalent representation of the MVDR beamformer offering computational and numerical advantages over a direct implementation in its original form.

The hearing aid may comprise antenna and transceiver circuitry allowing a wireless link to an entertainment device (e.g. a TV-set), a communication device (e.g. a telephone), a wireless microphone, or another hearing aid, etc. The hearing aid may thus be configured to wirelessly receive a direct electric input signal from another device. Likewise, the hearing aid may be configured to wirelessly transmit a direct electric output signal to another device. The direct electric input or output signal may represent or comprise an audio signal and/or a control signal and/or an information signal.

In general, a wireless link established by antenna and transceiver circuitry of the hearing aid can be of any type. The wireless link may be a link based on near-field communication, e.g. an inductive link based on an inductive coupling between antenna coils of transmitter and receiver parts. The wireless link may be based on far-field, electromagnetic radiation. Preferably, frequencies used to establish a communication link between the hearing aid and the other device is below 70 GHz, e.g. located in a range from 50 MHz to 70 GHz, e.g. above 300 MHz, e.g. in an ISM range above 300 MHz, e.g. in the 900 MHz range or in the 2.4 GHz range or in the 5.8 GHz range or in the 60 GHz range (ISM=Industrial, Scientific and Medical, such standardized ranges being e.g. defined by the International Telecommunication Union, ITU). The wireless link may be based on a standardized or proprietary technology. The wireless link may be based on Bluetooth technology (e.g. Bluetooth Low-Energy technology), or Ultra WideBand (UWB) technology.

The hearing aid may be configured to operate in different modes, e.g. a normal mode and one or more specific modes, e.g. selectable by a user, or automatically selectable. A mode of operation may be optimized to a specific acoustic situation or environment. A mode of operation may include a low-power mode, where functionality of the hearing aid is reduced (e.g. to save power), e.g. to disable wireless communication, and/or to disable specific features of the hearing aid.

Use:

In an aspect, use of an STM detection test unit and/or a hearing system as described above, is moreover provided. Use may be provided in a system comprising one or more hearing aids (e.g. hearing instruments), headsets, earphones, active ear protection systems, etc.

A method:

In an aspect, a method is furthermore provided by the present application.

The method comprises presenting a first probe stimulus to one ear of a user.

The method comprises presenting a second probe stimulus to another ear of the user.

The first probe stimulus and/or the second probe stimulus are presented by a stimulus generation unit comprising at least one output unit.

The method comprises determining a modulation-detection threshold of the user, by an analysis unit.

The modulation-detection threshold is determined in response to presenting the probe stimuli.

Presenting the probe stimuli refers to presenting a first probe stimulus to one ear of a user and a second probe stimulus to another ear of the user.

The method comprises generating each of the first probe stimulus and the second probe stimulus based on a carrier signal with a spectro-temporal modulation added, by the stimulus generation unit.

The spectro-temporal modulation of the first probe stimulus is different from the spectro-temporal modulation of the second probe stimulus.

The method may further comprise comparing the modulation-detection threshold of the user in response to the stimuli with a reference modulation-detection threshold.

The method may further comprise comparing the modulation-detection threshold of the user in response to the stimuli in each of the plurality of modes with a reference modulation-detection threshold of a reference mode.

The method may comprise determining a difference value between the modulation-detection threshold of the user and the reference modulation-detection threshold.

The reference modulation-detection threshold may be determined as indicated in the 'STM detection test unit' section above.

The method may further comprise adjusting/configuring signal processing parameters of a hearing aid of the user based on the determined difference value between the modulation-detection threshold of the user and the reference modulation-detection threshold.

The method provides measures of a hearing aid user's ability to select their better ear, as well as their ability to integrate temporally, spectrally, and spectro-temporally sparse information across the two ears. The availability and degree of these abilities represents crucial information for the prescription of suitable signal processing parameters of the hearing aid.

For example, one type of hearing aid signal processing is bilateral beamforming, where both hearing aids are used jointly to obtain improved spatial beamforming in order to increase speech intelligibility.

In case the user has poor binaural integration abilities, a bilateral beamforming would be parametrized aggressively, so that the resulting signal played to the two ears is identical, which would remove all acoustic aspects necessary for binaural processing. Depending on the user, this is often not desirable and therefore needs to be balanced by adding direct sound, which in turn compromises the efficacy of the beamforming for speech intelligibility improvement. Therefore, if a user is found to have very poor binaural integration abilities, aggressive bilateral beamforming may be just the right choice as it helps with speech intelligibility and likely comes at zero cost given the user's limited binaural integration ability.

It is intended that some or all of the structural features of the STM detection test unit and/or the system described above, in the 'detailed description of embodiments' or in the claims can be combined with embodiments of the method, when appropriately substituted by a corresponding process and vice versa. Embodiments of the method have the same advantages as the corresponding STM detection test unit and/or system.

A computer readable medium or data carrier:

In an aspect, a tangible computer-readable medium (a data carrier) storing a computer program comprising program code means (instructions) for causing a data processing system (a computer) to perform (carry out) at least some (such as a majority or all) of the (steps of the) method described above, when said computer program is executed on the data processing system is furthermore provided by the present application.

A computer program:

A computer program (product) comprising instructions which, when the program is executed by a computer, cause the computer to carry out (steps of) the method described is furthermore provided by the present application.

A data processing system:

In an aspect, a data processing system comprising a processor and program code means for causing the processor to perform at least some (such as a majority or all) of the steps of the method described above, is furthermore provided by the present application.

An APP:

In a further aspect, a non-transitory application, termed an APP, is furthermore provided by the present disclosure. The APP comprises executable instructions configured to be executed on an auxiliary device to implement a user interface for an STM detection test unit or a hearing system described above. The APP may be configured to run on cellular phone, e.g. a smartphone, or on another portable device allowing communication with said hearing system.

BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:

FIG. 1 shows an exemplary STM detection test unit according to the present application.
FIG. 2 shows exemplary stimuli design for testing better-ear selection according to the present application.
FIG. 3 shows exemplary stimuli design for testing temporal integration across ears according to the present application.
FIG. 4 shows exemplary stimuli design for testing spectral integration across ears according to the present application.
FIG. 5 shows exemplary stimuli design for testing spectro-temporal integration across ears according to the present application.

The figures are schematic and simplified for clarity, and they just show details which are essential to the understanding of the disclosure, while other details are left out. Throughout, the same reference signs are used for identical or corresponding parts.

Further scope of applicability of the present disclosure will become apparent from the detailed description given hereinafter. However, it should be understood that the detailed description and specific examples, while indicating preferred embodiments of the disclosure, are given by way of illustration only. Other embodiments may become apparent to those skilled in the art from the following detailed description. The scope of the invention is defined by the appended claims.

DETAILED DESCRIPTION OF EMBODIMENTS

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

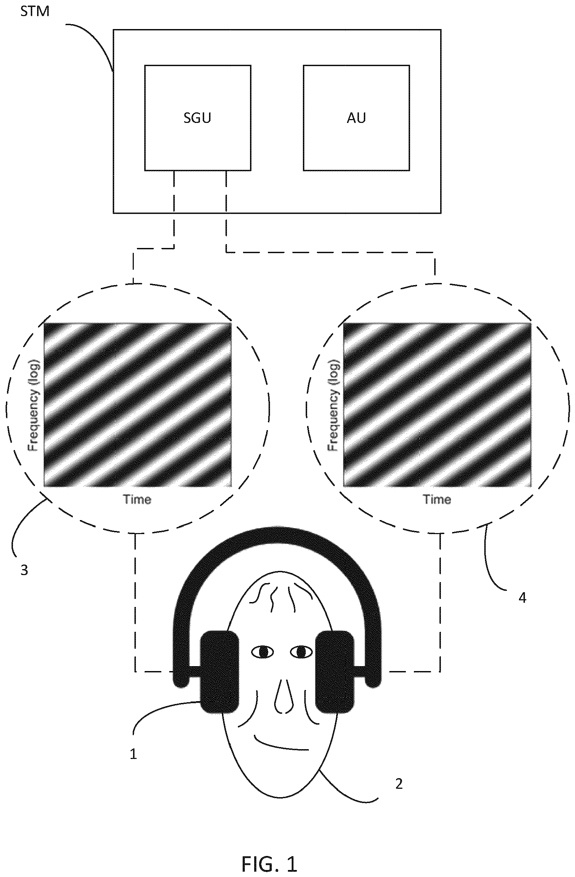

FIG. 1 shows an exemplary STM detection test unit according to the present application.

In FIG. 1, it is shown that the STM detection test unit ('STM') may comprise a stimulus generation unit SGU and an analysis unit AU.

The stimulus generation unit SGU may comprise at least one output unit. In FIG. 1, it is shown that the STM detection test unit ('STM') may further comprise a headset 1. The headset 1 may comprise a first output transducer and a second output transducer of the output unit.

The stimulus generation unit SGU may be configured to present a first probe stimulus to one of the ears of a user 2 via the first output transducer and to present a second probe stimulus to another ear of the user 2 via the second output transducer.

The stimulus generation unit SGU may be configured to generate each of the first probe stimulus and the second probe stimulus based on a carrier signal provided with a spectro-temporal modulation or without a modulation. In FIG. 1, it is shown that the first probe stimulus 3 and the second probe stimulus 4 may comprise similar spectro-temporal modulation. However, the first probe stimulus 3 (e.g. the spectro-temporal modulation) may also be different from the second probe stimulus 4 (e.g. the spectro-temporal modulation).

The analysis unit AU may be configured to determine, in response to presenting the probe stimuli, a modulation-detection threshold of the user 2. The modulation-detection threshold may be determined based on detected psychophysical or electrophysiological responses of the user 2 (not shown in FIG. 1).

It is contemplated that one or both of the stimulus generation unit SGU and an analysis unit AU may be incorporated into the headset. It is also contemplated that the STM detection test unit ('STM') may comprise an auxiliary device, e.g. a mobile device or stationary device, wired or wirelessly connected to the remainder features of the STM detection test unit ('STM'), and that the auxiliary device may control the stimulus generation unit SGU and/or the analysis unit AU.

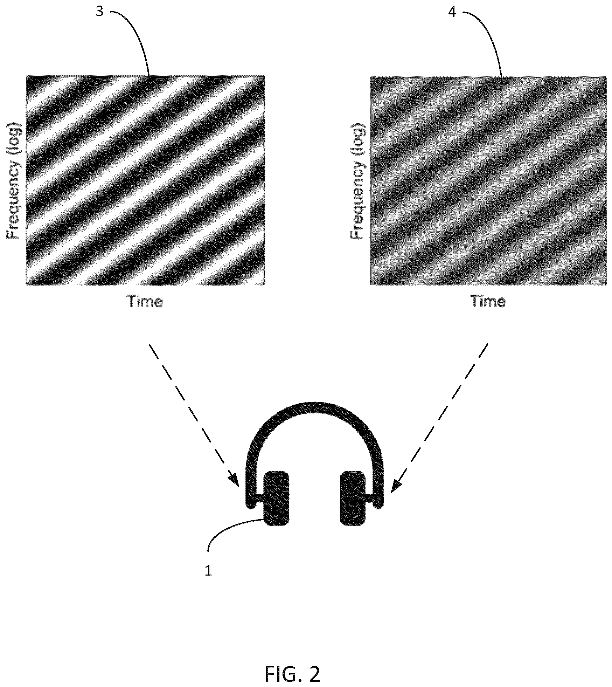

FIG. 2 shows exemplary stimuli design for testing better-ear selection according to the present application.

In FIG. 2, a first probe stimulus 3 is presented to one ear of a user (not shown) and a second probe stimulus 4 is presented to another ear of the user (not shown). Both stimuli 3,4 comprise a spectro-temporal modulation. However, the STM detection test unit ('STM') may be configured to operate in a plurality of different modes and in the mode of FIG. 2, the spectro-temporal modulation of the first probe stimulus 3 is different from the spectro-temporal modulation of the second probe stimulus 4.

The mode of FIG. 2 relates to a better-ear selection. In FIG. 2, it is assessed whether a user is able to select the information provided by the long-term better ear to optimize performance. This may be achieved by varying the degree of modulation of the stimuli between the two ears.

For example, in this mode, the modulation of the second probe stimulus 4 may be reduced compared to the modulation of the first probe stimulus 3 (e.g. by a fixed amount), making the ear receiving the first probe stimulus 3 the better ear in that trial. In the next trial, the modulation of the first probe stimulus 3 may be reduced compared to the modulation of the second probe stimulus 4. The difference value in performance between the described mode and a reference mode (e.g. the standard ACT test providing a reference modulation-detection threshold, or other) reveals the level of difficulty induced by having only one reliable ear signal in any given trial (instead of two). In the case, the user manages optimal better-ear selection, the difference value should be low or zero. In the case, the user only manages suboptimal better-ear selection, the difference value should be higher (>zero).

Additionally, the difference value may be compared to an average difference value measured for a group of young normal hearing listeners/subjects (normative data) at a similar test mode (i.e. at a similar mode of the STM detection test unit). Thereby, it may be determined whether the user's ability to use the better ear is decreased/impaired.

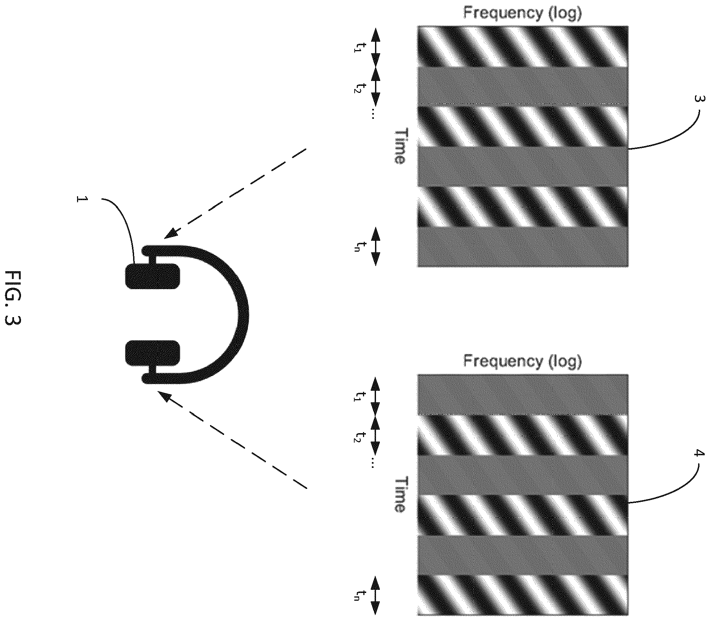

FIG. 3 shows exemplary stimuli design for testing temporal integration across ears according to the present application.

In FIG. 3, a first probe stimulus 3 is presented to one ear of a user (not shown) and a second probe stimulus 4 is presented to another ear of the user (not shown). Both stimuli 3,4 comprise a spectro-temporal modulation. However, in the mode of FIG. 3, the spectro-temporal modulation of the first probe stimulus 3 differs temporally from the spectro-temporal modulation of the second probe stimulus 4.

In FIG. 3, it is shown that the first probe stimulus 3 provides a spectro-temporal modulation at a first time interval $t_1$, but does not provide a modulation at a second time interval $t_2$, etc., alternating up to a time interval $t_n$. The second probe stimulus 4, on the other hand, provides no modulation at a first time interval $t_1$, but provides a spectro-temporal modulation at a second time interval $t_2$, etc., alternating up to a time interval $t_n$.

The mode of FIG. 3 relates to a temporal integration across the ears of the user. The mode of FIG. 3 measures the user's ability to integrate temporally sparse information across two ears to optimize performance. This may be achieved by splitting up the spectro-temporal modulation pattern imposed on the carrier signal in short time windows and modulating the noise only in the left- or in the right-ear signal in any given time window, in an alternating fashion over time. As a result, a perfect combination of the two temporally sparse but complementary spectro-temporal modulation patterns across ears reveals the full spectro-temporal modulation pattern. The difference value in performance between the described test and a reference mode (e.g. the standard ACT test providing a reference modulation-detection threshold, or other) reveals the increase in difficulty induced by having only one reliable ear signal in any given time window. In the case of optimal integration by the user, the performance should be the same and the difference value low or zero; in the case of suboptimal integration by the user, the performance will be lower and the difference value higher (>zero).

Additionally, the difference value may be compared to an average difference value measured for a group of young normal hearing listeners/subjects (normative data) at a similar test mode (i.e. at a similar mode of the STM detection test unit). Thereby, it may be determined whether the user's ability to integrate across ears is decreased/impaired.

Other modulation patterns may be contemplated.

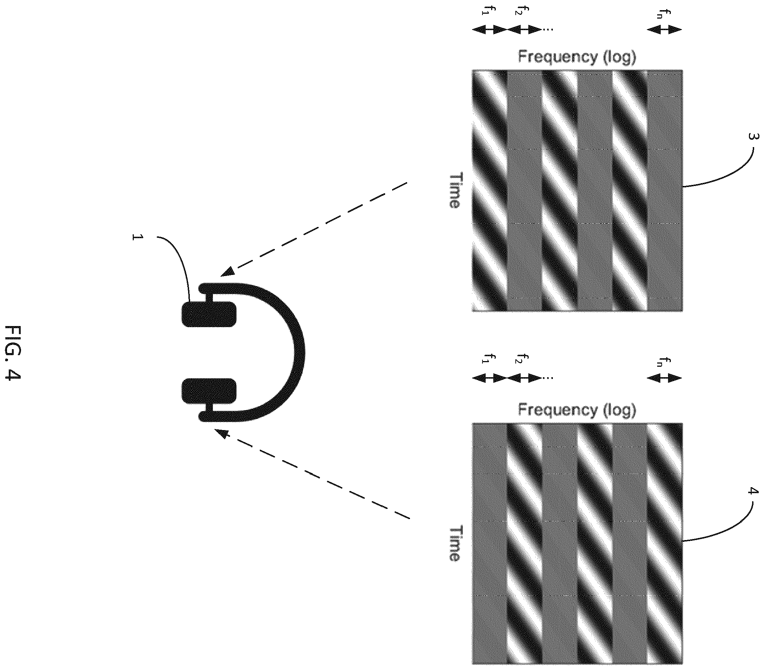

FIG. 4 shows exemplary stimuli design for testing spectral integration across ears according to the present application.

In FIG. 4, a first probe stimulus 3 is presented to one ear of a user (not shown) and a second probe stimulus 4 is presented to another ear of the user (not shown). Both stimuli 3,4 comprise a spectro-temporal modulation. However, in the mode of FIG. 4, the spectro-temporal modulation of the first probe stimulus 3 differs spectrally from the spectro-temporal modulation of the second probe stimulus 4.

In FIG. 4, it is shown that the first probe stimulus 3 provides a spectro-temporal modulation at a first frequency band $f_1$, but does not provide a modulation at a second frequency band $f_2$, etc., alternating up to a frequency band $f_n$. The second probe stimulus 4, on the other hand, provides no modulation at a first frequency band $f_1$, but provides a spectro-temporal modulation at a second frequency band $f_2$, etc., alternating up to a frequency band $f_n$.

The mode of FIG. 4 relates to spectral integration across ears. The mode of FIG. 4 measures the user's ability to integrate spectrally sparse information across the two ears to optimize performance. This is achieved by splitting up the spectro-temporal modulation pattern imposed on the carrier signal in auditory-inspired frequency bands and modulating the carrier signal only in the left- or in the right-ear signal in any given frequency band, in an alternating fashion across frequency. As a result, a perfect combination of the two spectrally sparse but complementary spectro-temporal modulation patterns across ears reveals the full spectro-temporal modulation pattern. The difference value in performance between the described mode and the reference mode (e.g. the standard ACT test providing a reference modulation-detection threshold, or other) reveals the increase in difficulty induced by having only one reliable ear signal in any given frequency band. In the case of optimal integration by the user, the performance should be the same and the difference value low or zero; in the case of suboptimal integration by the user, the performance should be lower and the difference value higher (>zero).

Additionally, the difference value may be compared to an average difference value measured for a group of young normal hearing listeners/subjects (normative data) at a similar test mode (i.e. at a similar mode of the STM detection test unit). Thereby, it may be determined whether the user's ability to integrate across ears is decreased/impaired.

Other modulation patterns may be contemplated.

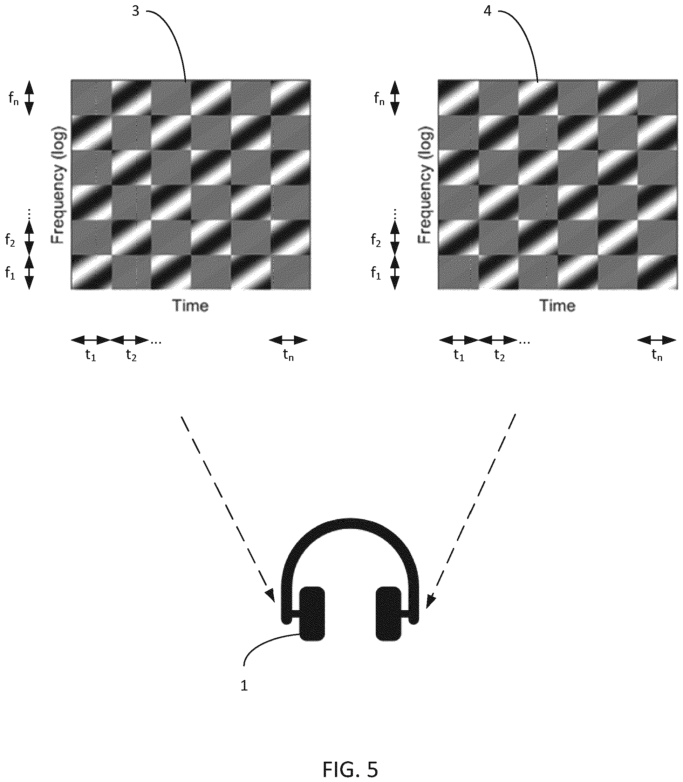

FIG. 5 shows exemplary stimuli design for testing spectro-temporal integration across ears according to the present application.

In FIG. 5, a first probe stimulus 3 is presented to one ear of a user (not shown) and a second probe stimulus 4 is presented to another ear of the user (not shown). Both stimuli 3,4 comprise a spectro-temporal modulation. However, in the mode of FIG. 5, the spectro-temporal modulation of the first probe stimulus 3 differs spectro-temporally from the spectro-temporal modulation of the second probe stimulus 4.

In FIG. 5, it is shown that the first probe stimulus 3 provides a spectro-temporal modulation at a first frequency band $f_1$ and first time interval $t_1$, and at a second frequency band $f_2$ and second time interval $t_2$, but does not provide a modulation at a second frequency band $f_2$ and first time interval $t_1$, and at a first frequency band $f_1$ and second time interval $t_2$, etc., alternating up to a frequency band $f_n$ and time interval $t_n$. The second probe stimulus 4, on the other hand, provides no modulation at a first frequency band $f_1$ and first time interval $t_1$, and at a second frequency band $f_2$

and second time interval $t_2$, but provides a spectro-temporal modulation at a second frequency band $f_2$ and first time interval $t_1$, and at a first frequency band $f_1$ and second time interval $t_2$, etc., alternating up to a frequency band $f_n$ and time interval $t_n$.

The mode of FIG. 5 relates to spectro-temporal integration across ears. The mode of FIG. 5 measures the user's ability to integrate spectro-temporally sparse information across the two ears to optimize performance. This is achieved by splitting up the spectro-temporal modulation pattern imposed on the carrier signal in short time windows and in auditory-inspired frequency bands. The resulting time-frequency units are selected in a checkerboard fashion such that the spectro-temporal modulation pattern in the left-ear signal is perfectly complementary to that in the right-ear signal. In other words, when there is modulation in a given time-frequency unit in the left ear, there is none in the right ear, and vice versa. As a result, a perfect combination of the two spectro-temporally sparse but complementary spectro-temporal modulation patterns across ears reveals the full spectro-temporal modulation pattern. The difference value in performance between the described test and the reference mode (e.g the standard ACT test providing a reference modulation-detection threshold, or other) reveals the increase in difficulty induced by having only one reliable ear signal in any given time-frequency unit. In the case of optimal integration by the user, the performance should be the same and the difference value low or zero; in the case of suboptimal integration by the user, the performance should be lower and the difference value (>zero).

Additionally, the difference value may be compared to an average difference value measured for a group of young normal hearing listeners/subjects (normative data) at a similar test mode (i.e. at a similar mode of the STM detection test unit). Thereby, it may be determined whether the user's ability to integrate across ears is decreased/impaired.

Other modulation patterns may be contemplated.

In FIGS. 2-5, only the stimulus design and not the features of the STM detection test unit ('STM') are shown, but it is obviously foreseen that some or all of the features of the STM detection test unit ('STM') may also be included.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element, but an intervening element may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method are not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may applied to other aspects.

<u>REFERENCES</u>

[1] Bernstein, J. G. W., Danielsson, H., Hällgren, M., Stenfelt, S., Rönnberg, J., & Lunner, T. (2016). Spectrotemporal Modulation Sensitivity as a Predictor of Speech-Reception Performance in Noise With Hearing Aids. Trends in Hearing, 20(0).

[2] Bernstein, J. G. W., Mehraei, G., Shamma, S., Gallun, F. J., Theodoroff, S. M., & Leek, M. R. (2013). Spectro-temporal Modulation Sensitivity as a Predictor of Speech Intelligibility for Hearing-Impaired Listeners. Journal of the American Academy of Audiology, 24(4), 293-306.

[3] Mehraei, G., Gallun, F. J., Leek, M. R., & Bernstein, J. G. (2014). Spectrotemporal modulation sensitivity for hearing-impaired listeners: Dependence on carrier center frequency and the relationship to speech intelligibility. The Journal of the Acoustical Society of America, 136(1), 301-316.

[4] Zaar, J., Simonsen, L. B., Behrens, T., Dau, T., & Laugesen, S. (2018). Towards a clinically viable spectro-temporal modulation test. IHCON Poster B3-P-15. International Hearing Aid Research Conference, Lake Tahoe, USA.

[5] Zaar, J., Simonsen, L. B., Behrens, T., Dau, T., & Laugesen, S. (2020). Investigating the relationship between spectro-temporal modulation detection, aided speech perception, and directional noise reduction preference in hearing-impaired listeners. Proceedings of the International Symposium on Auditory and Audiological Research, 7, 181-188. Retrieved from https://proceedings.isaar.eu/index.php/isaarproc/article/view/2019-22

[6] Best, V., Mason, C. R., Kidd, G., Iyer, N., & Brungart, D. S. (2015). Better-ear glimpsing in hearing-impaired listeners. The Journal of the Acoustical Society of America, 137(2), EL213-EL219.

[7] Brungart, D. S., & Iyer, N. (2012). Better-ear glimpsing efficiency with symmetrically-placed interfering talkers. The Journal of the Acoustical Society of America, 132(4), 2545-2556.

[8] Glyde, H., Buchholz, J., Dillon, H., Best, V., Hickson, L., & Cameron, S. (2013). The effect of better-ear glimpsing on spatial release from masking. The Journal of the Acoustical Society of America, 134(4), 2937-2945.

[9] Rana, B., & Buchholz, J. M. (2016). Better-ear glimpsing at low frequencies in normal-hearing and hearing-impaired listeners. The Journal of the Acoustical Society of America, 140(2), 1192-1205.

## Claims

1. Spectro-temporal modulation (STM) detection test unit comprising:

   - a stimulus generation unit comprising at least one output unit configured to simultaneously present a first probe stimulus to one ear of a user and present a second probe stimulus to another ear of the user,
   - a headset (1) comprising a first output transducer of the output unit for presenting the first probe stimulus (3) to one of the ears of the user, and a second output transducer of the output unit for presenting the second probe stimulus (4) to the other of the ears of the user,
   - an analysis unit configured to determine, in response to presenting the probe stimuli, a modulation-detection threshold of the user,
   - where the stimulus generation unit being configured to generate each of the first probe stimulus and the second probe stimulus based on a carrier signal with a spectro-temporal modulation added, and
   - where the spectro-temporal modulation of the first probe stimulus is different from the spectro-temporal modulation of the second probe stimulus.

2. STM detection test unit according to claim 1, wherein the STM detection test unit is configured to operate in a plurality of different modes, where each mode is **characterized by** the spectro-temporal modulation of the first probe stimulus being different from the spectro-temporal modulation of the second probe stimulus.

3. STM detection test unit according to any one of the preceding claims, wherein the spectro-temporal modulation of the first probe stimulus being different from the spectro-temporal modulation of the second probe stimulus comprises:

   - the degree and/or occurrence of the spectro-temporal modulation of the first probe stimulus being different from the degree and/or occurrence of the spectro-temporal modulation of the second probe stimulus.

4. STM detection test unit according to any one of the preceding claims, wherein the analysis unit being configured to compare the modulation-detection threshold of the user in response to the stimuli with a reference modulation-detection threshold.

5. STM detection test unit according to claim 4, wherein comparing the modulation-detection thresholds comprises:

   - the analysis unit being configured to determine a difference value between the modulation-detection threshold of the user and the reference modulation-detection threshold.

6. STM detection test unit according to any one of claims 4-5, wherein a reference modulation-detection threshold comprises one of:

   - a modulation-detection threshold of the user determined in response to presenting the combined probe stimuli of the chosen mode of the STM detection test unit to both ears of the user,
   - a modulation-detection threshold of a normal-hearing subject determined in response to presenting the combined probe stimuli of the chosen mode of the STM detection test unit to both ears of the normal-hearing subject, or
   - a modulation-detection threshold of the user determined in response to presenting similar sparse spectro-temporally modulated probe stimuli to both ears of the user.

7. STM detection test unit according to any one of the preceding claims, wherein generating the first probe stimulus and the second probe stimulus comprises:

  - the stimulus generation unit being configured to modulate the carrier signal of each of the first probe stimulus and the second probe stimulus by a modulator signal with an adjustable modulation depth parameter, where the modulation depth parameter determines the degree of modulation.

8. STM detection test unit according to any one of the preceding claims, wherein generating the first probe stimulus and the second probe stimulus comprises:

  - the stimulus generation unit being configured to reduce the modulation depth parameter of either the first probe stimulus or the second probe stimulus by a modulation reduction parameter.

9. STM detection test unit according to any one of the preceding claims, wherein generating the first probe stimulus and the second probe stimulus comprises:

  - the stimulus generation unit being configured to provide a mask on the modulator signal of each of the first probe stimulus and the second probe stimulus.

10. STM detection test unit according to any one of the preceding claims, wherein the STM detection test unit comprising one or more electrodes, and where the STM detection test unit is configured to determine the modulation-detection threshold of the user based on detecting a physiological response of the user by the one or more electrodes.

11. STM detection test system comprising:

  - an STM detection test unit according to any one of the claims 1-10, and
  - an auxiliary device.

12. STM detection test system according to claim 11, wherein the auxiliary device is a hearing aid, the hearing aid adapted for being located at or in an ear of a hearing aid user, or for being fully or partially implanted in the head of a hearing aid user, where the hearing aid comprising:

  - an input unit for receiving an input sound signal from an environment of a hearing aid user and providing at least one electric input signal representing said input sound signal, and
  - a processing unit comprising signal processing parameters to provide processed versions of said at least one electric input signal,
  - where the signal processing parameters are configured by at least the difference value between the modulation-detection threshold of the user and the reference modulation-detection threshold, according to any one of claims 5-10.

13. Method comprising:

  - simultaneously presenting a first probe stimulus to one ear of a user and presenting a second probe stimulus to another ear of the user, by a stimulus generation unit comprising at least one output unit via a headset comprising a first output transducer and a second output transducer of the output unit,
  - determining, in response to presenting the probe stimuli, a modulation-detection threshold of the user, by an analysis unit,
  - generating each of the first probe stimulus and the second probe stimulus based on a carrier signal with a spectro-temporal modulation added, by the stimulus generation unit,
  - where the spectro-temporal modulation of the first probe stimulus is different from the spectro-temporal modulation of the second probe stimulus.

14. Method according to claim 13, wherein the method further comprising:

  - comparing the modulation-detection threshold of the user in response to the stimuli with a reference modulation-detection threshold, and determining a difference value between the modulation-detection threshold of the user and the reference modulation-detection threshold.

**15.** Method according to any one of claims 13-14, wherein the method further comprises:

- adjusting signal processing parameters of a hearing aid of the user according to claim 12 based on the determined difference value between the modulation-detection threshold of the user and the reference modulation-detection threshold.

**Patentansprüche**

**1.** Spektro-temporale Modulationsdetektionstesteinheit (STM-Detektionstesteinheit), umfassend:

- eine Stimuluserzeugungseinheit, umfassend mindestens eine Ausgabeeinheit, die dazu konfiguriert ist, gleichzeitig einen ersten Sondenstimulus an einem Ohr eines Benutzers darzulegen und einen zweiten Sondenstimulus an einem anderen Ohr des Benutzers darzulegen,
- ein Headset (1), umfassend einen ersten Ausgangswandler der Ausgabeeinheit zum Darlegen des ersten Sondenstimulus (3) an eines der Ohren des Benutzers und einen zweiten Ausgangswandler der Ausgabeeinheit zum Darlegen des zweiten Sondenstimulus (4) an das andere der Ohren des Benutzers,
- eine Analyseeinheit, die dazu konfiguriert, als Reaktion auf das Darlegen der Sondenstimuli eine Modulationsdetektionsschwelle des Benutzers zu bestimmen,
- wobei die Stimuluserzeugungseinheit dazu konfiguriert ist, jeden des ersten Sondenstimulus und des zweiten Sondenstimulus auf Grundlage eines Trägersignals, dem eine spektro-temporale Modulation hinzugefügt ist, zu erzeugen, und
- wobei sich die spektro-temporale Modulation des ersten Sondenstimulus von der spektro-temporalen Modulation des zweiten Sondenstimulus unterscheidet.

**2.** STM-Detektionstesteinheit nach Anspruch 1, wobei die STM-Detektionstesteinheit dazu konfiguriert ist, in einer Vielzahl von unterschiedlichen Modi betrieben zu werden, wobei jeder Modus **dadurch gekennzeichnet ist, dass** sich die spektro-temporale Modulation des ersten Sondenstimulus von der spektro-temporalen Modulation des zweiten Sondenstimulus unterscheidet.

**3.** STM-Detektionstesteinheit nach einem der vorhergehenden Ansprüche, wobei der Unterschied der spektro-temporale Modulation des ersten Sondenstimulus von der spektro-temporalen Modulation des zweiten Sondenstimulus Folgendes umfasst:

- das Ausmaß und/oder das Auftreten der spektro-temporalen Modulation des ersten Sondenstimulus unterscheidet sich von dem Ausmaß und/oder dem Auftreten der spektro-temporalen Modulation des zweiten Sondenstimulus.

**4.** STM-Detektionstesteinheit nach einem der vorhergehenden Ansprüche, wobei die Analyseeinheit dazu konfiguriert ist, die Modulationsdetektionsschwelle des Benutzers als Reaktion auf die Stimuli mit einer Referenzmodulationsdetektionsschwelle zu vergleichen.

**5.** STM-Detektionstesteinheit nach Anspruch 4, wobei das Vergleichen der Modulationsdetektionsschwellen Folgendes umfasst:

- die Analyseeinheit ist dazu konfiguriert, einen Differenzwert zwischen der Modulationsdetektionsschwelle des Benutzers und der Referenzmodulationsdetektionsschwelle zu bestimmen.

**6.** STM-Detektionstesteinheit nach einem der Ansprüche 4-5, wobei eine Referenzmodulationsdetektionsschwelle eines der Folgenden umfasst:

- eine Modulationsdetektionsschwelle des Benutzers, die als Reaktion auf ein Darlegen der kombinierten Sondenstimuli des ausgewählten Modus der STM-Detektionstesteinheit an beide Ohren des Benutzers bestimmt wird,
- eine Modulationsdetektionsschwelle eines normal hörenden Individuums, die als Reaktion auf ein Darlegen der kombinierten Sondenstimuli des ausgewählten Modus der STM-Detektionstesteinheit an beide Ohren des normal hörenden Individuums bestimmt wird, oder
- eine Modulationsdetektionsschwelle des Benutzers, die als Reaktion auf ein Darlegen ähnlicher spärlicher spektro-temporaler modulierter Sondenstimuli an beide Ohren des Benutzers bestimmt wird.

7. STM-Detektionstesteinheit nach einem der vorhergehenden Ansprüche, wobei das Erzeugen des ersten Sondenstimulus und des zweiten Sondenstimulus Folgendes umfasst:

   - die Stimuluserzeugungseinheit ist dazu konfiguriert, das Trägersignal von jedem des ersten Sondenstimulus und des zweiten Sondenstimulus durch ein Modulatorsignal mit einem einstellbaren Modulationstiefenparameter zu modulieren, wobei der Modulationstiefenparameter das Ausmaß der Modulation bestimmt.

8. STM-Detektionstesteinheit nach einem der vorhergehenden Ansprüche, wobei das Erzeugen des ersten Sondenstimulus und des zweiten Sondenstimulus Folgendes umfasst:

   - die Stimuluserzeugungseinheit ist dazu konfiguriert, den Modulationstiefenparameter entweder des ersten Sondenstimulus oder des zweiten Sondenstimulus um einen Modulationsreduktionsparameter zu reduzieren.

9. STM-Detektionstesteinheit nach einem der vorhergehenden Ansprüche, wobei das Erzeugen des ersten Sondenstimulus und des zweiten Sondenstimulus Folgendes umfasst:

   - die Stimuluserzeugungseinheit ist dazu konfiguriert, eine Maske auf dem Modulatorsignal von jedem des ersten Sondenstimulus und des zweiten Sondenstimulus bereitzustellen.

10. STM-Detektionstesteinheit nach einem der vorhergehenden Ansprüche, wobei die STM-Detektionstesteinheit eine oder mehrere Elektroden umfasst und wobei die STM-Detektionstesteinheit dazu konfiguriert ist, die Modulationsdetektionsschwellen des Benutzers auf Grundlage eines Detektierens einer physiologischen Reaktion des Benutzers durch die eine oder die mehreren Elektroden zu bestimmen.

11. STM-Detektionstestsystem, umfassend:

    - eine STM-Detektionstesteinheit nach einem der Ansprüche 1-10, und
    - eine Hilfsvorrichtung.

12. STM-Detektionstestsystem nach Anspruch 11, wobei die Hilfsvorrichtung ein Hörgerät ist, wobei das Hörgerät dazu angepasst ist, sich an oder in einem Ohr eines Hörgerätebenutzers zu befinden oder vollständig oder teilweise in dem Kopf eines Hörgerätebenutzers implantiert zu sein, wobei das Hörgerät Folgendes umfasst:

    - eine Eingangseinheit zum Empfangen eines Eingangsschallsignals aus einer Umgebung eines Hörgerätebenutzers und zum Bereitstellen mindestens eines elektrischen Eingangssignals, welches das Eingangsschallsignal darstellt, und
    - eine Verarbeitungseinheit, umfassend Signalverarbeitungsparameter, um verarbeitete Versionen des mindestens einen elektrischen Eingangssignals bereitzustellen,
    - wobei die Signalverarbeitungsparameter mindestens durch den Differenzwert zwischen der Modulationsdetektionsschwelle des Benutzers und der Referenzmodulationsdetektionsschwelle nach einem der Ansprüche 5-10 konfiguriert sind.

13. Verfahren, umfassend:

    - gleichzeitiges Darlegen eines ersten Sondenstimulus an einem Ohr eines Benutzers und Darlegen eines zweiten Sondenstimulus an einem anderen Ohr des Benutzers durch eine Stimuluserzeugungseinheit, die mindestens eine Ausgabeeinheit umfasst, über ein Headset, das einen ersten Ausgangswandler und einen zweiten Ausgangswandler der Ausgabeeinheit umfasst,
    - Bestimmen, als Reaktion auf das Darlegen der Sondenstimuli, einer Modulationsdetektionsschwelle des Benutzers durch eine Analyseeinheit,
    - Erzeugen von jedem des ersten Sondenstimulus und des zweiten Sondenstimulus auf Grundlage eines Trägersignals, dem eine spektro-temporale Modulation hinzugefügt ist, durch die Stimuluserzeugungseinheit,
    - wobei sich die spektro-temporale Modulation des ersten Sondenstimulus von der spektro-temporalen Modulation des zweiten Sondenstimulus unterscheidet.

14. Verfahren nach Anspruch 13, wobei das Verfahren ferner Folgendes umfasst:

- Vergleichen der Modulationsdetektionsschwelle des Benutzers als Reaktion auf die Stimuli mit einer Referenzmodulationsdetektionsschwelle und Bestimmen eines Differenzwerts zwischen der Modulationsdetektionsschwelle des Benutzers und der Referenzmodulationsdetektionsschwelle.

**15.** Verfahren nach einem der Ansprüche 13-14, wobei das Verfahren ferner Folgendes umfasst:

- Einstellen von Signalverarbeitungsparametern eines Hörgeräts des Benutzers nach Anspruch 12 auf Grundlage des bestimmten Differenzwerts zwischen der Modulationsdetektionsschwelle des Benutzers und der Referenzmodulationsdetektionsschwelle.


**Revendications**

**1.** Unité d'essai de détection de modulation spectro-temporelle (STM) comprenant :

- une unité de génération de stimulus comprenant au moins une unité de sortie configurée pour présenter simultanément un premier stimulus de sonde à une oreille d'un utilisateur et présenter un second stimulus de sonde à une autre oreille de l'utilisateur,
- un casque (1) comprenant un premier transducteur de sortie de l'unité de sortie destiné à présenter le premier stimulus de sonde (3) à l'une des oreilles de l'utilisateur, et un second transducteur de sortie de l'unité de sortie destiné à présenter le second stimulus de sonde (4) à l'autre des oreilles de l'utilisateur,
- une unité d'analyse configurée pour déterminer, en réponse à la présentation des stimuli de sonde, un seuil de détection de modulation de l'utilisateur,
- où l'unité de génération de stimulus est configurée pour générer chacun du premier stimulus de sonde et du second stimulus de sonde sur la base d'un signal porteur avec une modulation spectro-temporelle ajoutée, et
- où la modulation spectro-temporelle du premier stimulus de sonde est différente de la modulation spectro-temporelle du second stimulus de sonde.

**2.** Unité d'essai de détection STM selon la revendication 1, ladite unité d'essai de détection STM étant configurée pour fonctionner dans une pluralité de modes différents, où chaque mode est **caractérisé par** la modulation spectro-temporelle du premier stimulus de sonde qui est différente de la modulation spectro-temporelle du second stimulus de sonde.

**3.** Unité d'essai de détection STM selon l'une quelconque des revendications précédentes, ladite modulation spectro-temporelle du premier stimulus de sonde différente de la modulation spectro-temporelle du second stimulus de sonde comprenant :

- le degré et/ou l'occurrence de la modulation spectro-temporelle du premier stimulus de sonde qui est différent du degré et/ou de l'occurrence de la modulation spectro-temporelle du second stimulus de sonde.

**4.** Unité d'essai de détection STM selon l'une quelconque des revendications précédentes, ladite unité d'analyse étant configurée pour comparer le seuil de détection de modulation de l'utilisateur en réponse aux stimuli avec un seuil de détection de modulation de référence.

**5.** Unité d'essai de détection STM selon la revendication 4, ladite comparaison des seuils de modulation-détection comprenant :

- l'unité d'analyse qui est configurée pour déterminer une valeur de différence entre le seuil de détection de modulation de l'utilisateur et le seuil de détection de modulation de référence.

**6.** Unité d'essai de détection STM selon l'une quelconque des revendications 4-5, un seuil de détection de modulation de référence comprenant un parmi :

- un seuil de détection de modulation de l'utilisateur déterminé en réponse à la présentation des stimuli de sonde combinés du mode choisi de l'unité d'essai de détection STM aux deux oreilles de l'utilisateur,
- un seuil de détection de modulation d'un sujet à audition normale déterminé en réponse à la présentation des stimuli de sonde combinés du mode choisi de l'unité d'essai de détection STM aux deux oreilles du sujet à audition normale, ou

- un seuil de détection de modulation de l'utilisateur déterminé en réponse à la présentation de stimuli de sonde modulés spectro-temporellement sporadiques similaires aux deux oreilles de l'utilisateur.

7. Unité d'essai de détection STM selon l'une quelconque des revendications précédentes, ladite génération du premier stimulus de sonde et du second stimulus de sonde comprenant :

- l'unité de génération de stimulus qui est configurée pour moduler le signal porteur de chacun du premier stimulus de sonde et du second stimulus de sonde par un signal de modulateur avec un paramètre de profondeur de modulation réglable, où le paramètre de profondeur de modulation détermine le degré de modulation.

8. Unité d'essai de détection STM selon l'une quelconque des revendications précédentes, ladite génération du premier stimulus de sonde et du second stimulus de sonde comprenant :

- l'unité de génération de stimulus qui est configurée pour réduire le paramètre de profondeur de modulation soit du premier stimulus de sonde, soit du second stimulus de sonde par un paramètre de réduction de modulation.

9. Unité d'essai de détection STM selon l'une quelconque des revendications précédentes, ladite génération du premier stimulus de sonde et du second stimulus de sonde comprenant :

- l'unité de génération de stimulus qui est configurée pour fournir un masque sur le signal de modulateur de chacun du premier stimulus de sonde et du second stimulus de sonde.

10. Unité d'essai de détection STM selon l'une quelconque des revendications précédentes, ladite unité d'essai de détection STM comprenant une ou plusieurs électrodes, et ladite unité d'essai de détection STM étant configurée pour déterminer le seuil de détection de modulation de l'utilisateur sur la base de la détection d'une réponse physiologique de l'utilisateur par la ou les électrodes.

11. Système d'essai de détection STM comprenant :

- une unité d'essai de détection STM selon l'une quelconque des revendications 1-10, et
- un dispositif auxiliaire.

12. Système d'essai de détection STM selon la revendication 11, ledit dispositif auxiliaire étant une prothèse auditive, ladite prothèse auditive étant adaptée pour être située au niveau ou dans une oreille d'un utilisateur de prothèse auditive, ou pour être totalement ou partiellement implantée dans la tête de l'utilisateur de prothèse auditive, où la prothèse auditive comprend :

- une unité d'entrée destinée à recevoir un signal sonore d'entrée en provenance d'un environnement d'un utilisateur de prothèse auditive et la fourniture d'au moins un signal d'entrée électrique représentant ledit signal sonore d'entrée, et
- une unité de traitement comprenant des paramètres de traitement de signal pour fournir des versions traitées dudit au moins un signal d'entrée électrique,
- où les paramètres de traitement du signal sont configurés par au moins la valeur de différence entre le seuil de détection de modulation de l'utilisateur et le seuil de détection de modulation de référence, selon l'une quelconque des revendications 5-10.

13. Procédé comprenant :

- la présentation simultanée d'un premier stimulus de sonde à une oreille d'un utilisateur et la présentation d'un second stimulus de sonde à une autre oreille de l'utilisateur, par une unité de génération de stimulus comprenant au moins une unité de sortie par l'intermédiaire d'un casque comprenant un premier transducteur de sortie et un second transducteur de sortie de l'unité de sortie,
- la détermination, en réponse à la présentation des stimuli de sonde, d'un seuil de détection de modulation de l'utilisateur, par une unité d'analyse,
- la génération de chacun du premier stimulus de sonde et du second stimulus de sonde sur la base d'un signal porteur avec une modulation spectro-temporelle ajoutée, par l'unité de génération de stimulus,
- où la modulation spectro-temporelle du premier stimulus de sonde est différente de la modulation spectro-temporelle du second stimulus de sonde.

**14.** Procédé selon la revendication 13, ledit procédé comprenant en outre :

- la comparaison du seuil de détection de modulation de l'utilisateur en réponse aux stimuli avec un seuil de détection de modulation de référence, et la détermination d'une valeur de différence entre le seuil de détection de modulation de l'utilisateur et le seuil de détection de modulation de référence.

**15.** Procédé selon l'une quelconque des revendications 13-14, ledit procédé comprenant en outre :

- le réglage des paramètres de traitement de signal d'une prothèse auditive de l'utilisateur selon la revendication 12 sur la base de la valeur de différence déterminée entre le seuil de détection de modulation de l'utilisateur et le seuil de détection de modulation de référence.

FIG. 1

FIG. 2

FIG. 3

FIG. 4

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Spectra-temporal modulation detection and its relation to speech perception in children with auditory processing disorder. **LOTFI YOUNES et al.** INTERNATIONAL JOURNAL OF PEDIATRIC OTORHINOLARYNGOLOGY. ELSEVIER, 03 January 2020, vol. 131
- Illusory spectrotemporal ripples created with binaurally correlated noise. **NASSIRI REZA et al.** THE JOURNAL OF THE ACOUSTICAL SOCIETY OF AMERICA. AMERICAN INSTITUTE OF PHYSICS FOR THE ACOUSTICAL SOCIETY OF AMERICA, 20 March 2008, vol. 123
- **BERNSTEIN, J. G. W ; DANIELSSON, H. ; HÄLLGREN, M. ; STENFELT, S. ; RÖNNBERG, J. ; LUNNER, T.** Spectrotemporal Modulation Sensitivity as a Predictor of Speech-Reception Performance in Noise With Hearing Aids. *Trends in Hearing,* 2016, vol. 20 (0
- **BERNSTEIN, J. G. W. ; MEHRAEI, G. ; SHAMMA, S. ; GALLUN, F. J. ; THEODOROFF, S. M. ; LEEK, M. R.** Spectrotemporal Modulation Sensitivity as a Predictor of Speech Intelligibility for Hearing-Impaired Listeners. *Journal of the American Academy of Audiology,* 2013, vol. 24 (4), 293-306
- **MEHRAEI, G. ; GALLUN, F. J. ; LEEK, M. R. ; BERNSTEIN, J. G.** Spectrotemporal modulation sensitivity for hearing-impaired listeners: Dependence on carrier center frequency and the relationship to speech intelligibility. *The Journal of the Acoustical Society of America,* 2014, vol. 136 (1), 301-316
- **ZAAR, J. ; SIMONSEN, L. B. ; BEHRENS, T ; DAU, T. ; LAUGESEN, S.** Towards a clinically viable spectro-temporal modulation test. IHCON Poster B3-P-15. *International Hearing Aid Research Conference, Lake Tahoe, USA,* 2018
- **ZAAR, J. ; SIMONSEN, L. B. ; BEHRENS, T. ; DAU, T. ; LAUGESEN, S.** Investigating the relationship between spectro-temporal modulation detection, aided speech perception, and directional noise reduction preference in hearing-impaired listeners. *Proceedings of the International Symposium on Auditory and Audiological Research,* 2020, vol. 7, 181-188, https://proceedings.isaar.eu/index.php/isaarproc/article/view/2019-22
- **BEST, V. ; MASON, C. R. ; KIDD, G. ; IYER, N. ; BRUNGART, D. S.** Better-ear glimpsing in hearing-impaired listeners. *The Journal of the Acoustical Society of America,* 2015, vol. 137 (2), EL213-EL219
- **BRUNGART, D. S. ; IYER, N.** Better-ear glimpsing efficiency with symmetrically-placed interfering talkers. *The Journal of the Acoustical Society of America,* 2012, vol. 132 (4), 2545-2556
- **GLYDE, H. ; BUCHHOLZ, J. ; DILLON, H. ; BEST, V. ; HICKSON, L. ; CAMERON, S.** The effect of better-ear glimpsing on spatial release from masking. *The Journal of the Acoustical Society of America,* 2013, vol. 134 (4), 2937-2945
- **RANA, B. ; BUCHHOLZ, J. M.** Better-ear glimpsing at low frequencies in normal-hearing and hearing-impaired listeners. *The Journal of the Acoustical Society of America,* 2016, vol. 140 (2), 1192-1205